(19) Europäisches Patentamt
European Patent Office
Office européen des brevets

(11) **EP 0 552 633 B1**

(12) **EUROPÄISCHE PATENTSCHRIFT**

(45) Veröffentlichungstag und Bekanntmachung des Hinweises auf die Patenterteilung:
**09.10.1996 Patentblatt 1996/41**

(51) Int Cl.6: **C07C 37/82**, C07C 39/07

(21) Anmeldenummer: **93100275.2**

(22) Anmeldetag: **11.01.1993**

(54) **Verfahren zur Trennung von m- und p-Kresol**

Process for the separation of m- and p-cresol

Procédé pour la séparation de m- et p-crésol

(84) Benannte Vertragsstaaten:
**BE CH DE FR GB IT LI NL**

(30) Priorität: **24.01.1992 DE 4201853**

(43) Veröffentlichungstag der Anmeldung:
**28.07.1993 Patentblatt 1993/30**

(73) Patentinhaber: **BAYER AG**
**51368 Leverkusen (DE)**

(72) Erfinder:
- **Buysch, Hans-Josef, Dr.**
  **W-4150 Krefeld (DE)**
- **Pentling, Ursula, Dr.**
  **W-4152 Kempen 3 (DE)**
- **Puppe, Lothar, Dr.**
  **W-5093 Burscheid (DE)**
- **Grosser, Rolf, Dr.**
  **W-5090 Leverkusen 1 (DE)**
- **Paul, Hanns-Ingolf, Dr.**
  **W-5000 Köln 80 (DE)**
- **Schnabel, Günter**
  **W-5632 Wermelskirchen 2 (DE)**

(56) Entgegenhaltungen:
GB-A- 2 058 772          US-A- 3 969 422

- CHEMICAL ABSTRACTS, vol. 96, no. 23, 1982, Columbus, Ohio, US; abstract no. 199259, Seite 631 ;Spalte 1 ;

## Beschreibung

Die vorliegende Erfindung beschreibt ein verbessertes Verfahren zur Trennung von m- und p-Kresol durch Adsorption an Zeolithen vom Faujasit-Typ, die mindestens ein Metallkation der ersten und/oder der zweiten Hauptgruppe des Periodensystems der Elemente (Mendelejew) enthalten, und wobei ein Desorptionsmittel mit einem Gehalt an aliphatischen und/oder cycloaliphatischen sekundären und/oder tertiären Alkoholen verwendet wird.

Während sich aus einem Gemisch von Kresol-Isomeren das o-Kresol recht gut destillativ abtrennen läßt, gelingt die Trennung von m- und p-Kresol mit Hilfe der Destillation nicht.

Die Trennung von Kresol-Isomeren mit Hilfe von Zeolithen als Adsorptionsmittel ist grundsätzlich bekannt (US 3.014.078; US 3 969 422). Hierbei sind vor allem weitporige Zeolithe verwendet worden, die auch mit Kationen ausgetauschte Faujasit-Typen umfassen. Als Desorptionsmittel werden dabei Phenole, primäre Alkohole und aromatische Kohlenwasserstoffe verwendet.

Sekundäre und tertiäre Alkohole gelten gemäß US '422 als nicht geeignete Desorptionsmittel. Bei einer solchen Verwendung von primären Alkoholen als Desorptionsmittel ist jedoch keine scharfe Isomerentrennung gewährleistet, d.h., ein Übergang an größeren Restanteilen eines Kresolisomeren in ein anderes kann nicht verhindert werden. So wird eine schärfere Trennung der Isomeren nur durch eine Vorbeladung des Adsorptionsmittels mit 6 bis 8 % Wasser erreicht. Da jedoch Wasser stärker gebunden wird als Kresole, wird die Adsorptionskapazität des Zeolithen hierdurch stark herabgesetzt. Die DE-OS 30 35 482 bezieht sich auf ein Verfahren zur Trennung isomerer Kresole unter Verwendung von aliphatischen Ketonen und Gemischen von aliphatischen Ketonen und Alkoholen. Es wird ein kontinuierlicher Prozeß als Beispiel angegeben, der Reinheiten für m- und p-Kresol von mehr als 99 % erzielen soll. Aufgrund der völlig unzureichenden Beschreibung der benutzten Apparatur läßt sich dieser Prozeß jedoch nicht nachvollziehen. So fehlt u.a. die Angabe der Anzahl und der Ausführung der Adsorptionskammern. Damit ist nicht allein die Menge des Adsorptionsmittels unbekannt, sondern auch die Anzahl der benötigten Trennstufen. Die Angaben reichen nicht aus, um die Konzentration der beiden Isomeren im Extrakt- und Raffinatstrom zu berechnen. Somit kann die angegebene Güte des Ergebnisses nicht sicher beurteilt werden.

In DE-OS 2 703 777 wird ein anderes Trennproblem behandelt, nämlich die Abtrennung der Xylenole von Kresolen. Auch hier werden Zeolithe eingesetzt, die jedoch unspezifisch Kationen aus unterschiedlichen Haupt- und Nebengruppen des Periodensystems der Elemente enthalten. Desorptionsmittel sind wiederum Alkohole neben Ketonen. Bei dieser Trennung wird vom unterschiedlichen Molekulargewicht, der unterschiedlichen Molekülgestalt und damit unterschiedlicher Polarität und Acidität Gebrauch gemacht. So erhält man beispielsweise (vergl. Versuch Nr. 9 in Tabelle 2 von DE-OS '777) aus einem Gemisch von 2,6-Xylenol mit den isomeren Kresolen und Phenol das 2,6-Xylenol als Raffinat in einer Reinheit von 99,9 %, während im Extrakt die drei isomeren Kresole nahezu quantitativ auftreten und somit in ihrem Verhältnis zueinander keine Änderung erfahren haben.

Der Erfindung liegt daher die Aufgabe zugrunde, geeignete Desorptionsmittel zur Verfügung zu stellen, die eine wirksame Trennung und Wiedergewinnung der m- und p- Kresol-Isomeren ermöglichen, wenn ein Gemisch derselben auf einen Faujasit-Zeolith als Adsorptionsmittel aufgegeben wird, wobei dieses Verfahren kontinuierlich oder diskontinuierlich durchgeführt werden kann.

Es wurde ein Verfahren zur Trennung von m- und p-Kresol unter Verwendung von Faujasit-Zeolithen als Adsorptionsmittel und anschließender Desorption gefunden, das dadurch gekennzeichnet ist, daß man als Desorptionsmittel ein Gemisch sekundärer und/oder tertiärer Alkohole mit einem oder mehreren Phenolen, aliphatischen gesättigten primären Alkoholen oder Ketonen und einen Zeolithen vom Faujasit-Typ einsetzt, der mindestens ein Kation eines Elements der ersten und/oder zweiten Hauptgruppe des Periodensystems der Elemente (Mendelejew) enthält.

Das erfindungsgemäße adsorptive Trennverfahren kann sowohl in der Gasphase als auch in der flüssigen Phase durchgeführt werden. Bevorzugt ist jedoch die Flüssigphasentrennung bei niederer Temperatur, um unerwünschte Nebenreaktionen des Beschickungsgemisches oder des Desorptionsmittels zu vermeiden.

Im erfindungsgemäßen Verfahren wird das p-Kresol bevorzugt adsorbiert.

Das erfindungsgemäße Verfahren ist demnach vor allem dadurch gekennzeichnet, daß man als Desorptionsmittel ein Gemisch sekundärer und/oder tertiärer Alkohole mit einem oder mehreren Phenolen, aliphatischen gesättigten primären Alkoholen oder Ketonen einsetzt. Solche Desorptionsmittel erfüllen die wichtigen Voraussetzungen eines guten Trenneffektes und einer guten Abtrennbarkeit von den isolierten Kresol-Isomeren.

Solche sekundären und tertiären (nicht primären) Alkohole lassen sich durch die Formel

$$R^1\text{-}C(R^3)OH\text{-}R^2 \qquad\qquad (I)$$

darstellen, in der

$R^1$, $R^2$ und $R^3$ unabhängig voneinander geradkettiges oder verzweigtes $C_1$-$C_6$-Alkyl bedeuten, wobei $R^3$ zusätzlich Wasserstoff bedeuten kann und wobei $R^1$ und $R^2$ gemeinsam weiterhin die Polyalkylengruppierung -(-$CH_2$-)$_n$- mit ganzzahligen Werten für n von 4 bis 7 bedeuten können.

Beispiele für erfindungsgemäß verwendbare sekundäre oder tertiäre Alkohole sind Isopropanol, Butanol-2, Pentanol-2, Pentanol-3, Hexanol-2, Hexanol-3, Heptanol-2, Heptanol-3, Heptanol-4, Octanol-2, Octanol-3, Octanol-4, sek.-Isopentylalkohol, tert.-Butanol, 2-Methyl-2-pentanol, 4-Methyl-2-pentanol, 3-Ethyl-3-pentanol, Cyclopentanol, Cyclohexanol, Cycloheptanol, Cyclooctanol, 1-Methyl-cyclopentanol, 1-Methyl-cyclohexanol.

In bevorzugter Weise werden sekundäre oder tertiäre Alkohole der Formel (I) eingesetzt, deren Reste $R^1$, $R^2$ und $R^3$ insgesamt 4-8 C-Atome enthalten; besonders bevorzugt sind solche sekundären oder tertiären Alkohole, deren Reste $R^1$, $R^2$ und $R^3$ insgesamt 4-7 C-Atome enthalten. In weiterhin bevorzugter Weise werden sekundäre Alkohole eingesetzt, in denen demnach $R^3$ Wasserstoff bedeutet. Ganz besonders bevorzugt sind Hexanol-2, Heptanol-2, Octanol-2 und 4-Methyl-2-pentanol.

Unter den Phenolen ist vor allen Dingen das nicht substituierte Phenol zu nennen. Einsetzbare Ketone haben insgesamt 3-8 C-Atome. Einsetzbare aliphatische gesättigte primäre Alkohole haben 3-9 C-Atome. Unter diesen zusätzlichen Lösungsmitteln sind die aliphatischen gesättigten primären Alkohole bevorzugt, beispielsweise Pentanol-1, Hexanol-1, Heptanol-1 und Octanol-1, ganz besonders bevorzugt Hexanol-1.

Mischungen von nicht primären mit primären Alkoholen unterliegen keiner wesentlichen Beschränkung, so daß sie erfindungsgemäß 0,1-100 Gew.-% an nicht primärem Alkohol enthalten können. Es ist hierbei erstaunlich, daß selbst so geringe Mengen an nicht primärem Alkohol wie 0,1-10 Gew.-%, ja sogar vorzugsweise 0,2-5 Gew.-% und noch besonders bevorzugt 0,5-3 Gew.-%, in den als Desorptionsmittel eingesetzten Mischungen erfindungsgemäß eine Trennung der Kresol-Isomeren ermöglichen.

Als Adsorptionsmittel für das erfindungsgemäße Verfahren eignen sich Faujasit-Zeolithe, die ein oder mehrere Kationen der ersten und/oder zweiten Hauptgruppe des Periodensystems der Elemente (Mendelejew) enthalten.

Bevorzugt sind hierbei die Kationen von Natrium, Kalium, Strontium und Barium, ganz besonders bevorzugt das Kation des Kaliums.

Das adsorptive Trennverfahren gemäß Erfindung wird bei einer Temperatur im Bereich von 0-350°C, bevorzugt von 20-250°C, besonders bevorzugt von 100-200°C und unter einem Druck im Bereich von 1-40 bar, bevorzugt 1-30 bar durchgeführt.

Es wurde insbesondere gefunden, daß sich bei Verwendung der genannten erfindungsgemäßen Desorptionsmittel die Selektivität der genannten Adsorptionsmittel für p-Kresol in Bezug auf andere Kresol-Isomere erhöht und daß sich die Desorptionsrate des p-Kresols von den genannten Adsorptionsmitteln verbessert, wodurch eine scharfe Isomerentrennung ermöglicht wird. Eine solche scharfe Trennung konnte bisher (US 3.969.422) nur durch Zusatz von Wasser erzielt werden, welches sich ungünstig auf die eingesetzten Zeolithe auswirkt und somit zu verringerten Standzeiten des Adsorptionsmittels führt. Die Erfindung stellt nun ein wasserfreies Verfahren zur Verfügung, das den Übergang von Restanteilen eines Kresol-Isomeren in ein anderes verhindert und so eine scharfe Trennung der Isomeren ermöglicht (Beispiel 1). In kontinuierlichen Verfahrensvarianten werden gemäß der Erfindung Reinheiten für m-Kresol von mehr als 99 % bei einer hohen Konzentration von mehr als 15 % im Raffinatstrom erreicht, wobei gleichzeitig auch für das p-Kresol Reinheiten von oberhalb 99 % erzielt werden (Beispiel 4).

Das Adsorptionsmittel befindet sich zweckmäßig in einer oder mehreren Kammern in Form einer dichten kompakten feststehenden Schicht, die alternativ mit dem Beschickungsgemisch und dem Desorptionsmittel in Kontakt gebracht wird. In der einfachsten Ausführung der Erfindung wird das Adsorbens in Form einer einzelnen statischen Schicht verwendet, wobei diese Verfahrensvariante nur halbkontinuierlich durchführbar ist. Bewegtbetten (moving bed) mit Gegenstrom oder simulierte Bewegtbettensysteme mit Gegenstrom haben jedoch eine viel größere Trenneffizienz als Systeme mit feststehenden Adsorbensbetten (fixed bed) und sind daher bevorzugt. Bei den Verfahren mit Bewegtbetten oder simulierten Bewegtbetten finden die Adsorption und die Desorption kontinuierlich statt, so daß eine kontinuierliche Erzeugung eines Extrakt- und eines Raffinatstromes als auch die kontinuierliche Zuführung von Beschickungs- und Desorptionsmittelströmen möglich ist. Eine bevorzugte Arbeitsweise zur Durchführung des erfindungsgemäßen Verfahrens ist die auf dem Fachgebiet als Gegenstromchromatographie mit simulierter Festbettbewegung bekannte Betriebsweise.

Zur Prüfung verschiedener Adsorptionsmittel und Desorptionsmittel mit einem bestimmten Beschickungsgemisch zwecks Messung der Adsorptionsmitteleigenschaften hinsichtlich ihrer Adsorptionskapazität, Selektivität und Austauschrate kann eine dynamische Prüfapparatur nach dem Prinzip einer Pulstestapparatur verwendet werden.

Eine solche Apparatur besteht beispielsweise aus einer thermostatisierbaren Adsorptionsmittelkammer mit Einlaß- und Auslaßteilen an den entgegengesetzten Enden der Kammer. Eine Druckregeleinrichtung gestattet den Betrieb der Kammer bei konstantem vorbestimmtem Druck.

Zur Bestimmung der Selektivitäten und anderer Kennwerte für verschiedene Adsorptionsmittelsysteme wird ein Impulstest in dieser Apparatur unter Anwendung der nachstehenden Arbeitsweise durchgeführt: Das Adsorptionsmittel wird bis zur Gleichgewichtseinstellung mit dem jeweiligen Desorptionsmittel gefüllt, indem das Desorptionsmittel durch die Adsorptionsmittelkammer geleitet wird. Danach wird ein Stoß oder Impuls der Beschickung, die bekannte Konzentrationen der Kresol-Isomeren enthält, sämtlich verdünnt im Desorptionsmittel, einige Minuten lang eingeführt. Zur Erleichterung der Untersuchung kann eine Indikatorverbindung, die nicht adsorbiert wird, in bekannter Konzentration in die Beschickung eingebracht werden. Der Fluß des Desorptionsmittels wird dann wieder aufgenommen und der Indikator, sofern ein solcher verwendet wird, und die Kresole werden wie bei der Flüssigkeits-Feststoff-Chromatographie eluiert. Der Ausfluß wird durch das in den Strom geschaltete chromatographische Analysengerät analysiert, und es werden Spuren der Umhüllungskurven entsprechender Komponentenspitzen entwickelt. Alternativ können Ausflußproben periodisch gesammelt und später gesondert durch Gaschromatographie analysiert werden.

Aus den Daten der chromatographischen Aufzeichnungen kann das Adsorptionsmittelverhalten hinsichtlich Kapazitätsindex für p-Kresol und Selektivität für p-Kresol in Bezug auf die anderen Kresole und Desorptionsrate des p-Kresols durch das Desorptionsmittel bestimmt und ausgewertet werden. Der Kapazitätsindex ist gekennzeichnet durch den Abstand zwischen der Mitte der p-Kresol-Spitzenhüllkurve und der Indikator-Spitzenhüllkurve oder einem anderen bekannten Bezugspunkt, z.B. dem Volumen des gepumpten Desorptionsmittels. Er wird ausgedrückt durch das während dieses Zeitintervalls gepumpte Volumen an Desorptionsmittel in Milliliter. Die relative Selektivität $\beta$ für p-Kresol in Bezug auf die anderen Kresole ist gekennzeichnet durch das Verhältnis des Abstandes zwischen der Mitte der p-Kresol-Spitzenhüllkurve und der Indikatorspitzenhüllkurve (oder einem anderen Bezugspunkt) zu den entsprechenden Abständen für die anderen Kresol-Isomeren. Die Austauschrate von p-Kresol mit dem Desorptionsmittel ist gekennzeichnet durch die Breite der p-Kresol-Spitzenhüllkurve bei Halbintensität. Je geringer die Breite des Peaks ist, desto größer ist die Desorptionsrate.

Die Beispiele zeigen Ergebnisse aus Pulstests, die mit einem K-Y-Zeolith durchgeführt wurden. Die Ergebnisse verdeutlichen den Vorteil der Abtrennung von p-Kresol aus einem Gemisch von Kresol-Isomeren durch Verwendung der erfindungsgemäßen Desorptionsmittel infolge Verbesserung der Desorptionsrate, die insgesamt zu einem verbesserten Verfahren führen. Die Beispiele dienen zur weiteren Veranschaulichung des erfindungsgemäßen Verfahrens, beschränken die Erfindung jedoch nicht auf diese gezeigten Veranschaulichungen.

Beispiel 1

Bei diesem Beispiel wurde das Adsorptionsmittel auf Basis eines K-Y-Zeoliths in die Adsorptionsmittelkammer einer Pulstestapparatur gefüllt, die während der gesamten Untersuchung bei einer konstanten Temperatur von 130°C gehalten wurde. An das Ausflußende der Kammer war ein Durchflußphotometer und ein automatischer Fraktionensammler angeschlossen. Es wurden Fraktionen im Abstand von 1 Minute genommen.

Zunächst wurde das Desorptionsmittel 1-Hexanol mit 2 Vol-% 2-Heptanol mit einem Fluß von 5,2 ml/min bei 130°C durch die Adsorptionsmittelkammer gepumpt. Dann wurde ein Beschickungsimpuls von 1 ml eines Gemisches aus m- und p-Kresol (70/30) über ein Probenventil in das System eingeschleift, und das Eluat wurde in der oben angegebenen Weise periodisch gesammelt. Die Ermittlung der quantitativen Zusammensetzung der einzelnen Proben erfolgte gaschromatographisch. Die Auftragung der ermittelten Flächen gegen ml Desorptionsmittel vom Zeitpunkt der Probenbeschickung ergab Hüllkurven für p- und m-Kresol aus denen Selektivitätswerte und Desorptionsraten abgeleitet wurden. Die Selektivität wurde berechnet durch Bestimmung der Nettoretentionsvolumina $V_p$ und $V_m$ von p- und m-Kresol gemäß nachfolgender Beziehung:

$$\alpha = (V_p - V_I)/(V_m - V_I),$$

wobei $V_I$ das Volumen der unretardierten Inertverbindung i-Octan bedeutet. In Vorversuchen wurde ein Volumen $V_I$ von 60 cm$^3$ ermittelt. Die Desorptionsrate ist gekennzeichnet durch die Halbwertsbreite der p-Kresol-Hüllkurve. Die Werte sind in Tabelle 1 aufgeführt.

Beispiel 2 (zum Vergleich, nicht erfindungsgemäß)

Beispiel 2 unterscheidet sich von Beispiel 1 dadurch, daß als Desorptionsmittel 1-Hexanol ohne Zusatz von 2-Heptanol verwendet wurde. Die Werte sind ebenfalls in Tabelle 1 aufgeführt.

Tabelle 1

|  | Retentionsvolumen p-/m-Kresol/ml | relat. Selektivität β p-/m-Kresol | Desorptionsrate p-Kresol/ml |
|---|---|---|---|
| Bsp.1 | 112/88 | 1,86 | 41 |
| Bsp.2 | 105/84 | 1,88 | 62 |

Die Ergebnisse der Tabelle 1 verdeutlichen die Vorteile der Kresol-Isomerentrennung durch Verwendung eines erfindungsgemäßen Desorptionsmittels. Die Desorptionsrate ist deutlich geringer und führt zu einer befriedigenden Kinetik des Trennprozesses ohne starkes Tailing der retardierten Komponente p-Kresol, wie dies im Beispiel 2 deutlich wird.

Beispiel 3

Dieser Versuch wurde unter Verwendung einer kontinuierlichen Adsorptionsvorrichtung durchgeführt. Diese Vorrichtung bestand aus 8 Adsorptionskammern von je 1188 cm$^3$ Volumen. Eingesetzt wurde K-Y-Zeolith, das Desorptionsmittel und das Isomerengemisch mit der Zusammensetzung aus Beispiel 1. Die Anlage wurde bei einer Temperatur von 130°C betrieben. Durch die Beschickungsleitungen wurden das Ausgangsgemisch (eine Lösung von 100 g m/p-Kresol in 100 g Desorptionsmittel) in einer Fließrate von 200 g/h und das Desorptionsmittel in einer Fließrate von 1200 g/h kontinuierlich zugeleitet. Die Taktzeit lag bei 1260 s. Der Kreislaufstrom betrug 2100 g/h.

Durch die Entnahmeleitungen für das Raffinat wurde m-Kresol mit einer Fließrate von 500 g/h in einer Konzentration von 15,0 Gew.-% und einer Isomerenreinheit von 99,0 % entnommen. Für das p-Kresol wurde im Extraktstrom eine Isomereneinheit von 90,0 % und eine Konzentration von 2,5 Gew.-% erzielt.

Beispiel 4

Dieser Versuch unterscheidet sich vom vorherigen dadurch, daß die Anlage bei einer Temperatur von 150°C und einem Druck von 3 bar betrieben wurde. Durch die Beschickungsleitungen wurden das Ausgangsgemisch (eine Lösung von 122 g m/p-Kresol in 123 g Desorptionsmittel wie in Beispiel 3) in einer Fließrate von 245 g/h und das Desorptionsmittel in einer Fließrate von 1255 g/h kontinuierlich zugeleitet. Die Taktzeit lag bei 1100 s. Der Kreislaufstrom betrug 2500 g/h. Durch die Entnahmeleitungen für das Raffinat wurde m-Kresol mit einer Fließrate von 600 g/h in einer Konzentration von 18,1 Gew.-% und einer Isomerenreinheit von 99,5 % entnommen. Die Isomereneinheit von p-Kresol im Extraktstrom beträgt ebenfalls 99,5 % bei einer Konzentration von 3 %.

**Patentansprüche**

1. Verfahren zur Trennung von m- und p-Kresol unter Verwendung von Faujasit-Zeolithen als Adsorptionsmittel und anschließender Desorption, dadurch gekennzeichnet, daß man als Desorptionsmittel ein Gemisch sekundärer und/oder tertiärer Alkohole mit einem oder mehreren Phenolen, aliphatischen gesättigten primären Alkoholen oder Ketonen und einen Zeolithen vom Faujasit-Typ einsetzt, der mindestens ein Kation eines Elements der 1. und/oder 2. Hauptgruppen des Periodensystems der Elemente (Mendelejew) enthält.

2. Verfahren nach Anspruch 1, dadurch gekennzeichnet, daß im Gemisch mindestens ein sekundärer oder tertiärer Alkohol der Formel

$$R^1\text{-}C(R^3)OH\text{-}R^2$$

eingesetzt wird, in der

R$^1$, R$^2$ und R$^3$ unabhängig voneinander geradkettiges oder verzweigtes $C_1$-$C_6$-Alkyl bedeuten, wobei R$^3$ zusätzlich Wasserstoff bedeuten kann und wobei R$^1$ und R$^2$ gemeinsam weiterhin die Polyalkylengruppierung -(-CH$_2$-)$_n$- mit ganzzahligen Werten für n von 4 bis 7 bedeuten können.

3. Verfahren nach Anspruch 2, dadurch gekennzeichnet, daß mindestens ein sekundärer oder tertiärer Alkohol im Gemisch eingesetzt wird, dessen Reste R$^1$, R$^2$ und R$^3$ insgesamt 4-8 C-Atome enthält.

4. Verfahren nach Ansprüchen 2 und 3, dadurch gekennzeichnet, daß im Gemisch mindestens ein sekundärer Alkohol eingesetzt wird.

5. Verfahren nach Anspruch 4, dadurch gekennzeichnet, daß im Gemisch mindestens ein sekundärer Alkohol aus der Gruppe von Hexanol-2, Heptanol-2, Octanol-2 und 4-Methyl-2-pentanol eingesetzt wird.

6. Verfahren nach Anspruch 1, dadurch gekennzeichnet, daß das Gemisch einen oder mehrere aliphatische gesättigte primäre Alkohole mit 3-9 C-Atomen enthält.

7. Verfahren nach Anspruch 6, dadurch gekennzeichnet, daß als aliphatischer gesättigter primärer Alkohol einer oder mehrere aus der Gruppe von Pentanol-1, Hexanol-1, Heptanol-1 und Octanol-1 eingesetzt wird (werden).

8. Verfahren nach Anspruch 6, dadurch gekennzeichnet, daß im Gemisch 0,1-10 Gew.-% an nicht primärem Alkohol vorhanden sind.

9. Verfahren nach Anspruch 1, dadurch gekennzeichnet, daß ausgetauschte Faujasit-Zeolithe mit mindestens einem Kation aus der Gruppe von Natrium, Kalium, Strontium und Barium eingesetzt werden.

## Claims

1. Process for the separation of m- and p-cresol, using faujasite zeolites as adsorbents, followed by desorption, characterised in that as desorbent use is made of a mixture of secondary and/or tertiary alcohols with one or more phenols, aliphatic saturated primary alcohols or ketones, and a zeolite of the faujasite type which contains at least one cation of an element of the first and/or second main group of the Periodic Table of the Elements (Mendeleev).

2. Process according to Claim 1, characterised in that in the mixture at least one secondary or tertiary alcohol of the formula

$$R^1\text{-}C(R^3)OH\text{-}R^2$$

is used, in which

$R^1$, $R^2$ and $R^3$, independently of each other, denote straight-chain or branched $C_1$-$C_6$-alkyl, where $R^3$ can additionally denote hydrogen and where $R^1$ and $R^2$ together can further denote the polyalkylene group $-(-CH_2-)_n-$ having integral values for n of 4 to 7.

3. Process according to Claim 2, characterised in that at least one secondary or tertiary alcohol, whose radicals $R^1$, $R^2$ and $R^3$ contain a total of 4-8 C atoms, is used in the mixture.

4. Process according to Claims 2 and 3, characterised in that at least one secondary alcohol is used in the mixture.

5. Process according to Claim 4, characterised in that at least one secondary alcohol selected from the group consisting of 2-hexanol, 2-heptanol, 2-octanol and 4-methyl-2-pentanol is used in the mixture.

6. Process according to Claim 1, characterised in that the mixture contains one or more aliphatic saturated primary alcohols having 3-9 C atoms.

7. Process according to Claim 6, characterised in that the aliphatic saturated primary alcohol(s) used is(are) one or more selected from the group consisting of 1-pentanol, 1-hexanol, 1-heptanol and 1-octanol.

8. Process according to Claim 6, characterised in that 0.1-10% by weight of non-primary alcohol is present in the mixture.

9. Process according to Claim 1, characterised in that exchanged faujasite zeolites having at least one cation selected from the group consisting of sodium, potassium, strontium and barium are used.

## Revendications

1. Procédé pour séparer le m- et le p-crésol par utilisation en tant qu'agents adsorbants de zéolites de type faujasite qu'on soumet ensuite à désorption, ce procédé se caractérisant en ce que l'on utilise en tant qu'agent désorbant un mélange d'alcools secondaires et/ou tertiaires avec un ou plusieurs phénols, des alcools aliphatiques saturés primaires ou des cétones, et une zéolite du type faujasite qui contient au moins un cation d'un élément du premier

EP 0 552 633 B1

et/ou du deuxième groupe principal de la classification périodique des éléments (Mendelejeff).

2. Procédé selon revendication 1, caractérisé en ce que l'on utilise dans le mélange au moins un alcool secondaire ou tertiaire de formule :

$$R^1-C(R^3)OH-R^2$$

dans laquelle
$R^1$, $R^2$ et $R^3$ représentent chacun, indépendamment les uns des autres, un groupe alkyle à chaîne droite ou ramifiée en $C_1$-$C_6$, $R^3$ pouvant en outre représenter l'hydrogène et $R^1$ et $R^2$ former ensemble un groupement polyalkylène -(-$CH_2$-)$_n$- dans lequel n est un nombre entier allant de 4 à 7.

3. Procédé selon revendication 2, caractérisé en ce que le mélange contient au moins un alcool secondaire ou tertiaire dont les substituants $R^1$, $R^2$ et $R^3$ contiennent au total de 4 à 8 atomes de carbone.

4. Procédé selon les revendications 2 et 3, caractérisé en ce que le mélange contient au moins un alcool secondaire.

5. Procédé selon revendication 4, caractérisé en ce que le mélange contient au moins un alcool secondaire du groupe formé par le 2-hexanol, le 2-heptanol, le 2-octanol et le 4-méthyl-2-pentanol.

6. Procédé selon revendication 1, caractérisé en ce que le mélange contient un ou plusieurs alcools aliphatiques saturés primaires en $C_3$-$C_9$.

7. Procédé selon revendication 6, caractérisé en ce que l'alcool aliphatique saturé primaire consiste en un ou plusieurs composés du groupe formé par le 1-pentanol, le 1-hexanol, le 1-heptanol et le 1-octanol.

8. Procédé selon revendication 6, caractérisé en ce que le mélange contient 0,1 à 10% en poids d'alcools non primaires.

9. Procédé selon revendication 1, caractérisé en ce que l'on utilise une zéolite du type faujasite qui a subi un échange de cations et contient au moins un cation du groupe du sodium, du potassium, du strontium et du baryum.